# EUROPEAN PATENT APPLICATION

(11) **EP 3 429 312 A1**
(43) Date of publication of application: **16.01.2019**
(21) Application number: 18171308.2
(22) Date of filing: 08.05.2018
(51) Int. Cl.: H05B 3/00, H05B 1/02, A61N 5/06, A61B 5/01

(54) **HEATING DEVICE WITH CONTACTLESS TEMPERATURE CONTROL**

(30) Priority: 12.07.2017 CN 201710564270
(71) Applicant: Kuno International co., Ltd., 10546 Taipei City (TW)
(72) Inventor: CHEN, Chien-Chen, Taipei City 10546 (TW)
(74) Representative: Zimmermann, Tankred Klaus

(57) **Abstract**

A heating device with a contactless temperature control includes a temperature sensor for sensing a surface temperature of an object, a control circuit connected to the temperature sensor, and a heating unit coupled to the control circuit. The control circuit controls the heating unit to generate thermal energy based on the surface temperature sensed by the temperature sensor. A more comfortable and safer environment can be provided by instantly sensing the surface temperature of a user's body and simultaneously controlling the heating unit to generate thermal energy. Moreover, the device can prevent the overheating, which may cause the secondary damage of the user. Besides, the device can also prevent the poor efficiency caused by the insufficient heating.

## Description

### BACKGROUND

### Technology Field

The present disclosure relates to a heating device and, in particular, to a heating device with a contactless temperature control that can change the heat energy according to the surface temperature of an object.

### Description of Related Art

As the progress of technology, the heating devices are widely applied in our lives. For example, when the weather becomes cold, people can be warmed by hot air machine or electric heater. In addition, the infrared light or the proper heating treatment device can be used to provide heat to and treat the affected area of the patient, such as a bums patient.

However, the conventional heating device only has some basic manual setup functions such as the functions of turning on/off, adjusting intensity, or adjusting the treating time, so that it cannot adjust the intensity and temperature based on the surface temperature of the human body in real time. Thus, the patients may experience the overcooling and overheating during the therapy. In particularly, when the heating device is used in the medical equipment, the irradiation time and intensity are usually set by professional or medical personnel in advance according to the situation of the patient. Thus, once the medical equipment is enabled, it will provide a light with the preset irradiation time and intensity regardless the actual situation of the patient during the irradiation procedure. Unfortunately, this operation may cause the issue of low temperature burns or secondary damage.

### SUMMARY

In view of the foregoing, an objective of the disclosure is to provide a heating device with a contactless temperature control that can change the thermal energy received by the user's body surface according to the surface temperature of a human body.

To achieve the above, the present disclosure provides a heating device with a contactless temperature control. The heating device includes a temperature sensor, a control circuit and a heating unit. The temperature sensor is configured for sensing a surface temperature of an object. The control circuit is connected to the temperature sensor, and the heating unit is coupled to the control circuit. The control circuit controls the heating unit to generate thermal energy based on the surface temperature sensed by the temperature sensor.

In one embodiment, the heating unit comprises a lamp for emitting the thermal energy and a lampshade connected to the lamp, and the control circuit controls the lamp to generate the thermal energy based on the surface temperature. The lamp is any one or two of a halogen lamp, a carbon lamp, and a ceramic lamp.

In one embodiment, the heating unit further comprises a tri-axial drive shaft connecting to the lampshade, and the control circuit controls the tri-axial drive shaft to move and controls the lamp to generate the thermal energy based on the surface temperature.

In one embodiment, the heating unit comprises a lamp for emitting the thermal energy, a lampshade connected to the lamp, and at least a reflective cover disposed inside the lampshade, and the control circuit controls the reflective cover to move and controls the lamp to generate the thermal energy based on the surface temperature.

In one embodiment, the heating unit comprises a plurality of lamps for emitting the thermal energy and a lampshade connected to the lamps, and the control circuit controls the lamps to generate the thermal energy based on the surface temperature.

In one embodiment, the heating device further includes an air temperature sensor connecting to the control circuit. The air temperature sensor is a camera configured for monitoring the object to obtain an image information. The image information includes a plurality of pixel signals and a plurality of temperature values corresponding to the pixel signals. The temperature sensor obtains a human body temperature range according to an environment temperature detected by the air temperature sensor. The temperature sensor determines whether the temperature values corresponding to the pixel signals of the image information are located within the human body temperature range and marks the pixel signals with the corresponding temperature values located within the human body temperature range, and the temperature sensor calculates with the temperature values corresponding to the marked pixel signals to obtain the surface temperature.

In one embodiment, the temperature sensor calculates an average value, a standard deviation, a deviation value, or any of combinations thereof to obtain the surface temperature.

In one embodiment, the temperature sensor is a camera. The temperature sensor monitors the object to obtain a plurality of images, and the images are combined to form an image information. The image information includes a plurality of pixel signals and a plurality of temperature values corresponding to the pixel signals, and the temperature sensor obtains the surface temperature according to a temperature variation of the image information.

In one embodiment, the heating device further includes a distance sensor connecting to the control circuit, and the distance sensor is configured to detect a distance between the object and the heating device.

In one embodiment, the heating device further includes a record module connecting to the control circuit for recording operation information of the temperature sensor, the control circuit and the heating unit.

As mentioned above, the heating device with a contactless temperature control of this disclosure can detect the surface temperature of a user in real time and control the heating unit to generate thermal energy at the same time. A more comfortable and safer environment can be provided by instantly sensing the surface temperature of a user's body and simultaneously controlling the heating unit to generate thermal energy. Moreover, the device can prevent the overheating, which may cause the secondary damage of the user. Besides, the device can also prevent the poor efficiency caused by the insufficient heating.

### BRIEF DESCRIPTION OF THE DRAWINGS

The disclosure will become more fully understood from the detailed description and accompanying drawings, which are given for illustration only, and thus are not limitative of the present disclosure, and wherein:
FIG. 1 is a schematic diagram showing a heating device with a contactless temperature control according to a first embodiment of the disclosure;
FIG. 2 is a schematic diagram showing a heating unit of the disclosure, wherein the heating unit has a reflective design;
FIG. 3 is a schematic diagram showing a heating unit of the disclosure, wherein the heating unit has a multiple lamp design;
FIG. 4 is a schematic diagram showing a heating unit of the disclosure, wherein the heating unit has a secondary reflective design;
FIG. 5 is a schematic diagram showing a heating device with a contactless temperature control according to a second embodiment of the disclosure;
FIG. 6 is a flow chart showing the operation steps of the heating device with a contactless temperature control according to the second embodiment of the disclosure;
FIG. 7 is a schematic graph showing the relation between the temperature and the surface temperature of the object;
FIG. 8 is a flow chart showing the detailed steps of the step S20 of FIG. 6; and
FIG. 9 is a flow chart showing additional detailed steps of the step S20 of FIG. 6.

### DETAILED DESCRIPTION OF THE DISCLOSURE

The present disclosure will be apparent from the following detailed description, which proceeds with reference to the accompanying drawings, wherein the same references relate to the same elements.

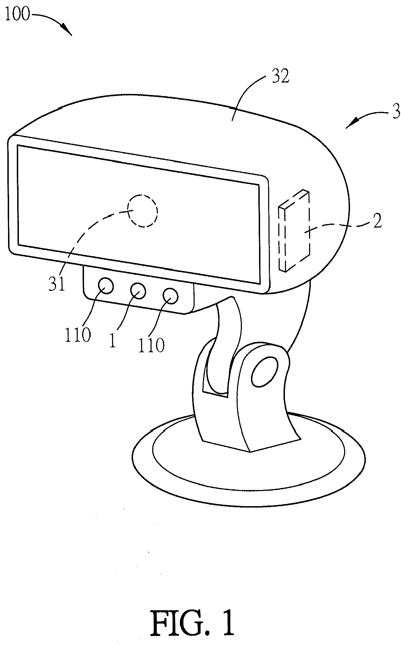

FIG. 1 is a schematic diagram showing a heating device 10 with a contactless temperature control according to a first embodiment of the disclosure. The heating device 100 can generate a thermal energy corresponding to the surface temperature of an object (e.g. a human body or an inorganic body). The heating device 100 can be applied to the heater or smart appliance with heating function, or to the medical equipment for treating the affected area of the burns patient. The applications fields of the heating device 100 is not limited. In this embodiment, the heating device 100 is applied to the medical equipment for treating the affected area of the patient. The heating device 100 includes a temperature sensor 1, a control circuit 2 connected to the temperature sensor 1, and a heating unit 3 coupled to the control circuit 2.

In this embodiment, the temperature sensor 1 is a contactless IR temperature sensor, and is used for detecting the surface temperature of the affected area of a patient. The control circuit 2 controls the operations of the circuit components. The heating unit 3 can communicate with the control circuit 2 by a wired or wireless method, so that the control circuit 2 can control the heating unit 3 to generate thermal energy according to the detected surface temperature for outputting the IR light to irradiate the affected area of the patient. Accordingly, the heating device 100 can detect the surface temperature of the affected area in real time and correspondingly control the intensity of the outputted IR ray (thermal energy). This configuration can prevent the overtime irradiation, which may cause burning or secondary damage, due to artificial missing.

In this embodiment, the heating unit 3 includes a lamp 31 and a lampshade 32 connected to the lamp 31. The lamp 31 can be a halogen lamp, a carbon lamp, or a ceramic lamp that can emit near/far infrared ray. In addition, the lamp 31 can include any two of the above light sources, so that the lamp 31 can output the light with two different spectrums, which can provide a fixed thermal energy (fixed spectrum) and a thermal energy of a specific temperature according to the surface temperature. Otherwise, the light with two different spectrums may both provide the thermal energies of a specific temperature according to the surface temperature, but are a near IR ray and a far IR ray. The lamp 31 can be modified based on different applications, and is not limited in the above. The lampshade 32 can collect the light emitted from the lamp 31 toward a specific direction (e.g. toward a human body).

To be noted, the control circuit 2 can control the exposure equivalent, frequency, power (watt) or duty cycle of the lamp 31 to change the temperature of the heating unit 3. Besides, the control circuit 2 can transform the AC driving input into a DC driving input for driving the heating unit 3. Of course, in other designs, the control circuit 2 does not need to perform the AC-DC transform, and can directly use the AC driving input to drive the heating unit 3. Moreover, the temperature sensor 1 can be a near/far IR temperature sensor or any sensor that can detect the surface temperature, and this disclosure is not limited.

The heating unit 3 has four designs including the tri-axial drive shaft type, reflective type, multiple lamp type, and secondary reflective type. In the tri-axial drive shaft type design, the heating unit 3 further includes a tri-axial drive shaft (not shown) connecting to the lampshade. The tri-axial drive design means that the lampshade 32 can be rotated about the X axis, Y axis and Z axis, so that the irradiating angle and range of the lamp 31 can be simultaneously controlled. Besides, it is also possible to add a proper mechanism to adjust the height of the lamp 31 for different applications. In some specific applications (e.g. the affected area can be a nonplanar surface such as the elbow or knee of the patient), the tri-axial drive design allows the control circuit 2 to control the heating unit 3 to rotate within a specific range based on the detected surface temperature of the affected area. For example, heating unit 3 can rotate in a circle (the radius is 5 cm) with the temperature sensor 1 as the center. Upon rotating, the heating unit 3 can simultaneously output the thermal energy to the affected area of the patient. This design can prevent the heat from focusing at the center of the elbow and can achieve a more uniform heating. Besides, this design can also have a broader irradiating range.

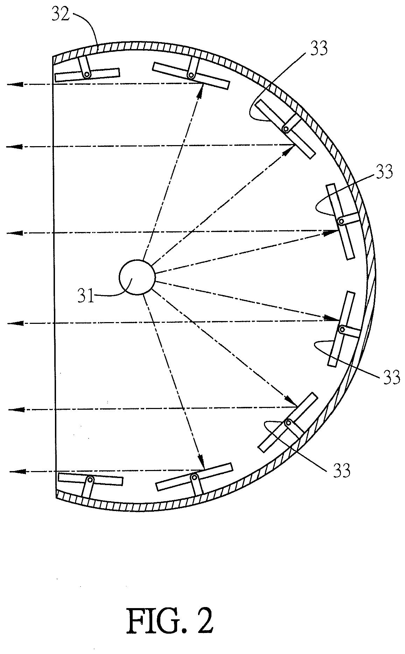

As shown in FIG. 2, in a reflective type design, the heating unit 3 further includes a reflective cover 33 disposed inside the lampshade 32, and the control circuit 2 controls the reflective cover 33 to move for providing a corresponding angle. Accordingly, if the affected area is a nonplanar surface such as the elbow or knee of the patient, the reflective design also allows the control circuit 2 to control the reflective angle of each reflective cover 33 based on the detected surface temperature of the affected area. This design can prevent the heat from focusing at the center of the elbow and can achieve a more uniform heating. Besides, this design can also have a broader irradiating range.

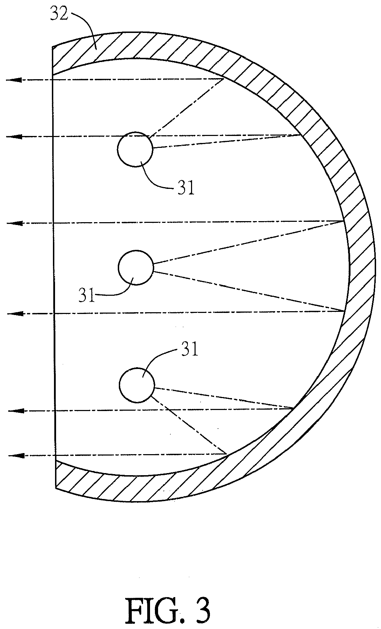

Referring to FIGS. 1 and 3, in the multiple lamp type design, the heating unit 3 includes a plurality of lamps 31 and a lampshade 32 connected to the lamps 31. Each lamp 31 can be a halogen lamp, a carbon lamp, or a ceramic lamp that can emit near/far infrared ray. In addition, each lamp 31 can include any two of the above light sources, so that the lamp 31 can output the light with two different spectrums. In this embodiment, the control circuit 2 can calculate the required heat equivalents of all positions based on the detected surface temperature of the affected area, and then control each lamp 31 of the heating unit 3 to output. Otherwise, the control circuit 2 may control to change the frequency, power (watt) or duty cycle of each lamp 31. When the temperature sensor 1 detects that the temperature of the center of a sensing point is higher than the temperature of the periphery of the center, the control circuit 2 can control to lower the temperature of the lamp 31 located at the center of all lamps 31 and to increase the temperature of the other lamps 31. This configuration can also achieve a more uniform heating.

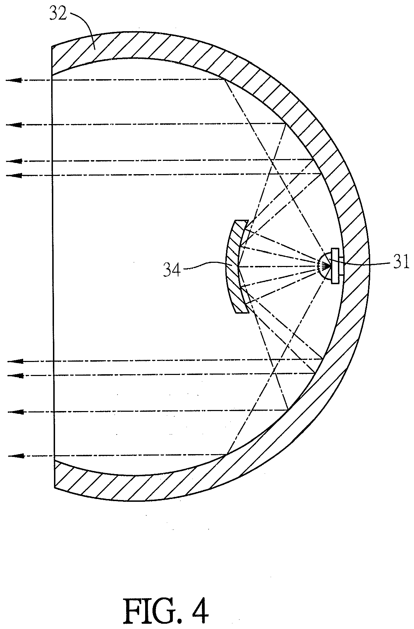

Referring to FIGS. 1 and 4, in the secondary reflective type design, the lamp 31 of the heating unit 3 is located in the lampshade 32, and the heating unit 3 further includes a reflective cover 34 corresponding to the lamp 31. The reflective cover 34 is configured to reflect the light emitted from the lamp 31, and the reflected light is reflected again by the lampshade 32 and then outputted. Accordingly, if the affected area is a nonplanar surface such as the elbow or knee of the patient, the secondary reflective design can also prevent the heat from focusing at the center of the elbow and can achieve a more uniform heating. Besides, this design can also have a broader irradiating range.

To be noted, the temperature sensor 1 can simultaneously change its detecting range corresponding to any of the above different heating units 3. In other words, the detecting point or detecting range of the temperature sensor 1 is not necessary to be fixed, and it can be changed based on the operation of the heating unit 3.

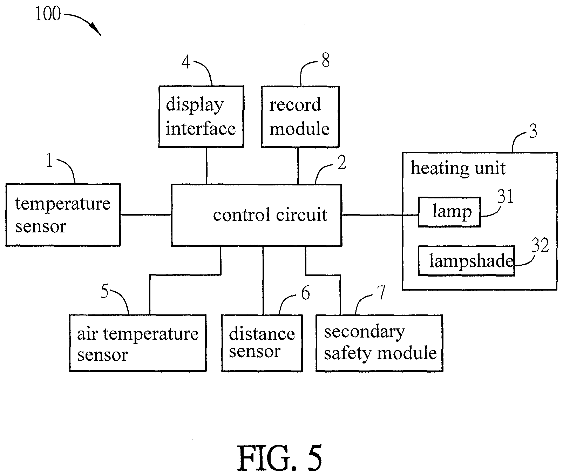

FIG. 5 is a schematic circuit diagram of a heating device 100 with a contactless temperature control according to a second embodiment of the disclosure. In this embodiment, the heating device 100 includes a temperature sensor 1, a control circuit 2 connected to the temperature sensor 1, a heating unit 3 coupled to the control circuit 2, a display interface 4 connected to the control circuit 2, and an air temperature sensor 5 connected to the control circuit 2.

In this embodiment, the temperature sensor 1 is a contactless IR camera, and is used for monitoring the affected area of a patient for detecting the surface temperature of the affected area of the patient. The operation of the temperature sensor 1 will be described hereinafter. The control circuit 2 and the heating unit 3 can be referred to the first embodiment, so the detailed descriptions thereof will be omitted. The display interface 4 can show the operation mode and the current status information of the heating device 100, such as the current temperature, irradiating time, and the likes. The air temperature sensor 5 is configured to detecting the environment temperature.

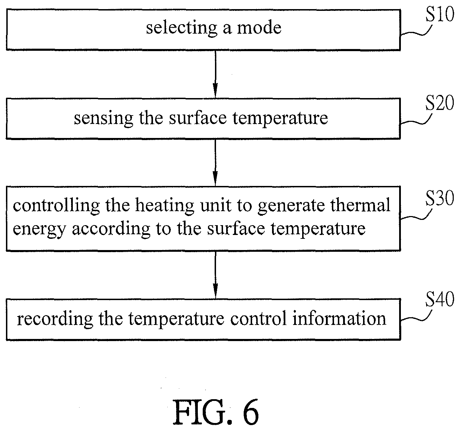

The operation of the heating device 100 of the second embodiment will be described hereinafter with reference to FIG. 6. To be noted, the operation of FIG. 6 can also be applied to the first embodiment.

In the step S10, when the heating device 100 is turned on, the user can select a desired mode, and the control circuit 2 can control the display interface 4 to show the selected mode. In this embodiment, the display interface 4 displays four modes for selection including "comfortable mode", "equivalent mode", "simple mode", and "therapy mode". In the "comfortable mode", the control circuit 2 controls the heating unit 3 to provide a comfortable temperature for the user (the user does not have to set a quantified temperature). In the "equivalent mode", the control circuit 2 controls the heating unit 3 to operate based on the operation (irradiating) time and temperature preset by the user. In the "simple mode", the control circuit 2 controls the heating unit 3 to operate based on the temperature preset by the user. In the "therapy mode", the control circuit 2 controls the output of the heating unit 3 based on the feedback of the surface temperature of the user, so that the user can be treated by a specific heating procedure according to the doctor prescription. The specific heating procedure includes the body surface temperature change, time, depth of radiation impact, radiation wavelength distribution, or a combination of any of the above. In this embodiment, the "therapy mode" is selected as an example.

To be noted, the user can select the desired mode by remote control, near-end control or automatic control. The remote control is to utilize a mobile device (e.g. a cell phone) to communicate with the heating device 100, and to select the desired mode by an application of the mobile device the button of the remote controller, or a voice control. In the voice control, the control circuit 2 is configured with a circuit for receiving the voice signal. In the near-end control, the user can directly operate the control keys 110 of the heating device 100 to select the desired mode. In the automatic control, the heating device 100 has a sensor (not shown) for automatically sensing the human body and selecting the desired mode according to the preset settings (e.g. four modes are shown in turn). Of course, the above embodiment is only for an illustration, and the type and number of the selectable modes and the selecting method are not limited to the above embodiment.

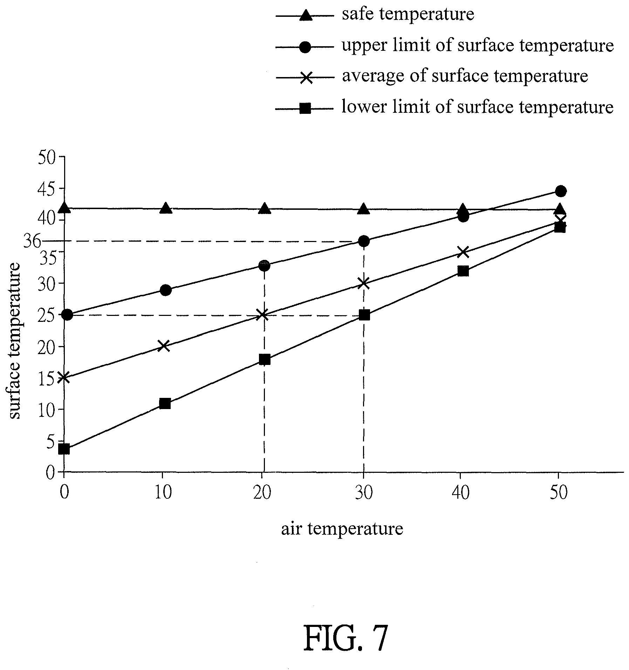

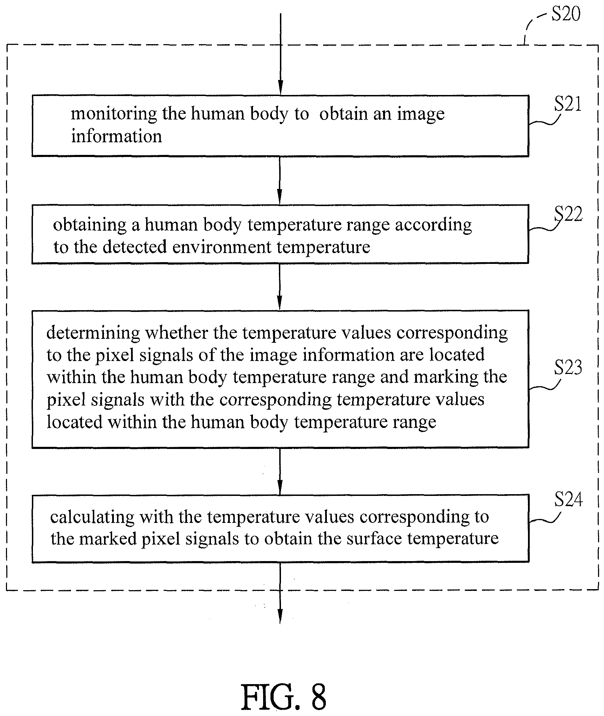

In the step S20, after selecting the desired mode (therapy mode), the temperature sensor 1 detects the surface temperature of the affected area of the patient. Referring to FIGS. 5, 7 and 8, the temperature sensor 1 can perform the temperature detection by the air temperature/body temperature model as shown in FIG. 7. At first, the temperature sensor 1 monitors the patient (human body) to obtain an image information (step S21). The image information includes a plurality of pixel signals and a plurality of temperature values corresponding to the pixel signals. In this embodiment, the amount of the pixel signals is determined based on the resolution of the camera. When the resolution of the camera is higher, more pixel signals can be obtained. The temperature sensor 1 obtains a human body temperature range according to an environment temperature detected by the air temperature sensor 5 in cooperated with the air temperature/body temperature model of FIG. 7 (step S22). For example, when the environment temperature detected by the air temperature sensor 5 is 30 degrees Celsius, the human body temperature range is 25∼36 degrees Celsius. Then, the temperature sensor 1 determines whether the temperature values corresponding to the pixel signals of the image information are located within the human body temperature range, and marks the pixel signals with the corresponding temperature values located within the human body temperature range (step S23). In other words, the image constructed by all of the marked pixel signals can represent the position of the human body. Finally, the temperature sensor 1 calculates with the temperature values corresponding to the marked pixel signals to obtain the surface temperature of the affected area of the patient (step S24).

To be noted, the values shown in the air temperature/body temperature model of FIG. 7 are for illustrations only, and they can be varied based on different environment or other factors. The temperature sensor 1 can calculate an average value, a standard deviation, or a deviation value of the temperature values of the marked pixel signals, or any of combinations thereof to obtain the surface temperature. Otherwise, the temperature sensor 1 can obtain the surface temperature by calculating the average of the top 20% of the temperature values of the marked pixel signals, and this disclosure is not limited. In addition, the temperature sensor 1 may be interfered by a heat source such as a light source or the sun, which can sufficiently increase the temperature average in the image information. Accordingly, in one embodiment, after the temperature sensor 1 obtains the image information, the pixel signal with the highest temperature value is removed, or the pixel signals with the top part (e.g. top 5%) of the temperature values are removed for enhancing the detection accuracy.

In the step S21, the temperature sensor 1 can take one or more photos (e.g. 20 photos) and combine the photos to form the image information. Then, the temperature sensor 1 can define the body position of the patient according to the temperature difference variation, and obtain the surface temperature according to the above-mentioned calculation method (e.g. the average, standard deviation, or the likes). Herein, the temperature difference variation can be obtained by calculating and determining whether the temperature difference variation of each pixel signal is less than (or greater than) a predetermined deviation value. This method can also obtain the surface temperature of the affected area of the patient, and this disclosure is not limited. In addition, the temperature difference variation can be applied to compare the temperature values before and after turning on/off the lamp 31 for clarifying the reflection issue. For example, upon turning on the lamp 31, the temperature will increase due to the light reflection. Moreover, the temperature difference variation can also be applied to the high heat source barrier, which has small variation before and after turning on the lamp 31 and cannot be easily heated. Besides, the temperature difference variation can also be applied to observe the relationship between the heating equivalent and heating rate of some pixel signals during the heating procedure of the lamp 31. This application can determine the surface property so as to realize whether the surface is an organic surface or which part the surface is in an organic body. Furthermore, this application can also determine whether to achieve the efficacy of vasodilation according to the variation of heating rate. In this case, the vasodilation can cause the increase of the heat dissipation rate, and the increase of the heat dissipation rate means the decrease of the heating rate.

In this embodiment, the heating device 100 further includes a distance sensor 6 connected to the control circuit 2 for detect a distance between the human body and the heating device 100. The distance sensor 100 can assist to increase the accuracy of the detection of the surface temperature. For example, when the distance sensor 6 detects that the distance is greater than a preset value, the temperature sensor 1 will stop the detection and the control circuit 2 control the display interface 4 to show an error or alert signal, thereby reminding the user that the heat device 100 has moved to far away. In addition, the distance sensor 6 can cooperate with an automatic control function to sense whether a human body is approached or not, and the preset mode selection function can be enabled for following operation.

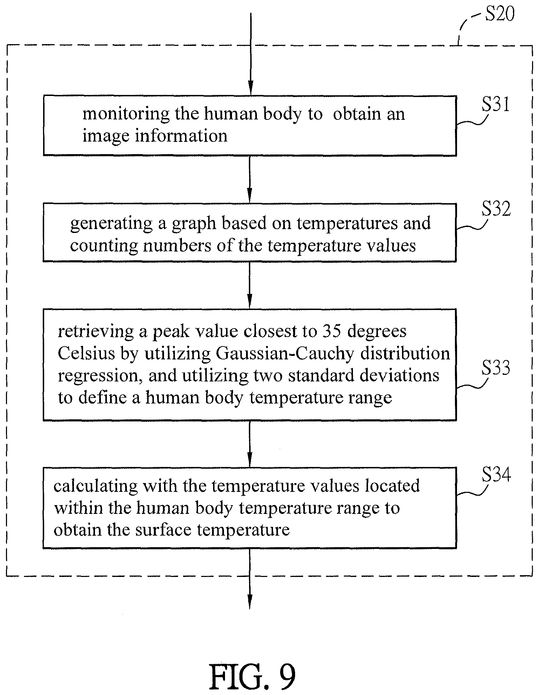

Referring to FIGS. 6 and 9, the temperature sensor 1 can obtain the human body temperature range by temperature screening. In practice, the temperature sensor 1 monitors the patient (human body) to obtain an image information (step S31). The image information includes a plurality of pixel signals and a plurality of temperature values or data corresponding to the pixel signals. Next, the temperature sensor 1 generates a graph based on temperatures (X-axis) and counting numbers (Y-axis) of the temperature data (step S32). When the environment temperature is in the normal range (no heat source approaching the human body temperature), the graph contains two obvious peaks. Then, a peak value closest to 35 degrees Celsius is retrieved by utilizing Gaussian-Cauchy distribution regression, and two standard deviations are utilized to define a human body temperature range (step S33). Finally, the temperature sensor 1 calculates with the temperature values located within the human body temperature range to obtain the surface temperature of the affected area of the patient (step S34).

In addition, the temperature sensor 1 can also obtain the human body temperature range by the distance/specific heat method. In more detailed, the temperature sensor 1 monitors the patient (human body) to obtain an image information, and the distance sensor 6 detects the distance between the heating device 100 and the center of the object. During a short period after turning on the lamp 31, the temperature increasing rate of each pixel is observed to determine whether the temperature increasing rate is located within the range of a normal human body temperature increasing rate, thereby obtaining the human body temperature range. This disclosure is not limited thereto.

Referring to FIGS. 5 and 6, in the step S30, the control circuit 2 controls the heating unit 3 to generate thermal energy according to the surface temperature detected by the temperature sensor 1. The control method of the control circuit 2 and the design of the heating unit 3 can be referred to the first embodiment, so the detailed descriptions thereof will be omitted.

In addition, the heating device 100 further includes a secondary safety module 7 connected to the control circuit 2 for preventing the heating unit 3 from generating overheated thermal energy. This configuration can protect the patient from bums once the major control system is out of control. In practice, once the temperature sensor 1 senses that the detected temperature is over a safety value, the secondary safety module 7 is enabled to control the heating unit 3 to generate the thermal energy within a safety temperature value or range. At the same time, the control circuit 2 stops controlling the heating unit 3, and the secondary safety module 7 takes advantages of the control mechanism until the patient (user) sets the heating device 100 again. The secondary safety module 7 can be an over-current/thermal shutdown protection circuit, an independent warning circuit, or any device that can prevent the heating unit 3 from generating the overheated thermal energy.

In the step S40, the heating device 100 further includes a record module 8 connecting to the control circuit 2 for recording the operation information of each circuit, such as the surface temperature detected by the temperature sensor 1, the operation time, modes, the schedule of irradiation equivalent, and the operation procedure of the lamp 31 (frequency, power, or duty cycle), so that the medical personnel can further control the treatment results of the patient. The record module 8 can record the information, and the control circuit 2 can control the display interface 4 to display the information. In addition, the information can be transferred from the record module 8 to another electronic device for performing a remote recording or analyzing.

As mentioned above, the heating device 100 with a contactless temperature control of this disclosure can detect the surface temperature of a user in real time and control the heating unit 3 to generate thermal energy (temperature) at the same time. This configuration can provide a more comfortable and safer environment. Moreover, when applying the heating device 100 in the medical application, the overheating, which may cause the secondary damage of the user, and the poor efficiency caused by the insufficient heating can be prevented so as to obtain a better treatment effect.

Although the disclosure has been described with reference to specific embodiments, this description is not meant to be construed in a limiting sense. Various modifications of the disclosed embodiments, as well as alternative embodiments, will be apparent to persons skilled in the art. It is, therefore, contemplated that the appended claims will cover all modifications that fall within the true scope of the disclosure.

## Claims

1. A heating device with a contactless temperature control, comprising:
a temperature sensor for sensing a surface temperature of an object;
a control circuit connected to the temperature sensor; and
a heating unit coupled to the control circuit, wherein the control circuit controls the heating unit to generate thermal energy based on the surface temperature sensed by the temperature sensor.

2. The heating device according to claim 1, wherein the heating unit comprises a lamp for emitting the thermal energy and a lampshade connected to the lamp, and the control circuit controls the lamp to generate the thermal energy based on the surface temperature.

3. The heating device according to claim 2, wherein the lamp is any one or two of a halogen lamp, a carbon lamp, and a ceramic lamp.

4. The heating device according to claim 2, wherein the heating unit further comprises a tri-axial drive shaft connecting to the lampshade, and the control circuit controls the tri-axial drive shaft to move and controls the lamp to generate the thermal energy based on the surface temperature.

5. The heating device according to claim 1, wherein the heating unit comprises a lamp for emitting the thermal energy, a lampshade connected to the lamp, and at least a reflective cover disposed inside the lampshade, and the control circuit controls the reflective cover to move and controls the lamp to generate the thermal energy based on the surface temperature.

6. The heating device according to claim 1, wherein the heating unit comprises a plurality of lamps for emitting the thermal energy and a lampshade connected to the lamps, and the control circuit controls the lamps to generate the thermal energy based on the surface temperature.

7. The heating device according to claim 1, further comprising an air temperature sensor connecting to the control circuit, wherein the air temperature sensor is a camera configured for monitoring the object to obtain an image information, the image information comprises a plurality of pixel signals and a plurality of temperature values corresponding to the pixel signals, the temperature sensor obtains a human body temperature range according to an environment temperature detected by the air temperature sensor, the temperature sensor determines whether the temperature values corresponding to the pixel signals of the image information are located within the human body temperature range and marks the pixel signals with the corresponding temperature values located within the human body temperature range, and the temperature sensor calculates with the temperature values corresponding to the marked pixel signals to obtain the surface temperature.

8. The heating device according to claim 7, wherein the temperature sensor calculates an average value, a standard deviation, a deviation value, or any of combinations thereof to obtain the surface temperature.

9. The heating device according to claim 1, wherein the temperature sensor is a camera, the temperature sensor monitors the object to obtain a plurality of images, the images are combined to form an image information, the image information comprises a plurality of pixel signals and a plurality of temperature values corresponding to the pixel signals, and the temperature sensor obtains the surface temperature according to a temperature variation of the image information.

10. The heating device according to claim 1, further comprising a distance sensor connecting to the control circuit, and the distance sensor is configured to detect a distance between the object and the heating device.

11. The heating device according to claim 1, further comprising a record module connecting to the control circuit for recording operation information of the temperature sensor, the control circuit and the heating unit.

12. The heating device according to claim 1, wherein the temperature sensor is a camera, the temperature sensor monitors the object to obtain an image information, the image information comprises a plurality of pixel signals and a plurality of temperature values corresponding to the pixel signals, the temperature sensor generates a graph based on temperatures and counting numbers of the temperature values, retrieves a peak value closest to 35 degrees Celsius by utilizing Gaussian-Cauchy distribution regression, and utilizes two standard deviations to define a human body temperature range, and the temperature sensor calculates with the temperature values located within the human body temperature range to obtain the surface temperature.
